# EUROPÄISCHE PATENTSCHRIFT

(11) **EP 1 018 645 B1**
(45) Veröffentlichungstag und Bekanntmachung des Hinweises auf die Patenterteilung: **12.05.2004**
(21) Anmeldenummer: 99123121.8
(22) Anmeldetag: 18.11.1999
(51) Int. Cl.: G01N 21/89

(54) **Vorrichtung zur Erkennung von Fremdstoffen in strangförmigem textilen Material**
Device for detection of foreign substances in strands of textile material
Appareil pour détection de matières étrangères d'un matériau textile en échevaux

(30) Priorität: 22.12.1998 DE 19859274
(43) Veröffentlichungstag der Anmeldung: 12.07.2000
(73) Patentinhaber: Saurer GmbH & Co. KG, 41069 Mönchengladbach (DE)
(72) Erfinder: Henze, Herbert, 41063 Mönchengladbach (DE); Birlem, Olav, 41366 Schwalmtal (DE)

(56) Entgegenhaltungen:
- EP-A- 0 399 945
- DE-A- 3 706 056
- US-A- 5 383 017
- US-A- 5 499 794
- US-A- 5 530 551

## Beschreibung

Die Erfindung betrifft eine Vorrichtung zur Erkennung von Fremdstoffen in strangförmigem textilen Material wie Faserbändern oder Garn, gemäß dem Oberbegriff des Anspruches 1.

Aus der EP 0 643 294 A1 ist es bekannt, zur Detektion von Fremdstoffen in einem textilen Material das Prüfgut zu beleuchten und das vom Prüfgut reflektierte Licht zu messen, wobei aus einer Änderung des reflektierten Lichts auf das Vorhandensein eines Fremdstoffes geschlossen wird. Zur Detektion von Fremdstoffen, die dunkler sind als das Prüfgut, wird das Prüfgut vor einem hellen Hintergrund auf einem Sensor abgebildet, zur Detektion von helleren Fremdstoffen vor einem dunklen Hintergrund. Als Beleuchtungselemente werden Leuchtdioden einer bestimmten Farbe, wie grün oder rot, verwendet. Für Leuchtdioden ist die Kurzbezeichnung "LED" gebräuchlich. Falls das von der Leuchtdiode emittierte Licht nicht ausreicht, um den Faden bei der verlangten Fadengeschwindigkeit ausreichend zu beleuchten, werden Beleuchtungselemente eingesetzt, die eine höhere Lichtmenge emittieren, wie Laser, Blitzlampen oder Glühlampen. Als alternative Maßnahme zur Verstärkung des emittierten Lichtes wird beschrieben, die Anzahl der LED zu vergrößern und mehrere LED einer bestimmten Farbe zu einer sogenannten LED-Multichipanordnung zusammenzufassen. Die Änderung des reflektierten Lichtes, aus der auf das Vorhandensein eines Fremdstoffes geschlossen wird, besteht in einer Änderung der Gesamthelligkeit des reflektierten Lichtes. Die in der EP 0 643 294 A1 beschriebene Vorrichtung weist jedoch Nachteile auf. Da Leuchtdioden einer bestimmten Farbe, wie grün oder rot, mit einem sehr schmalen Wellenlängenbereich arbeiten, kann es vorkommen, daß bestimmte Fremdstoffe keine oder keine ausreichende Helligkeitsänderung verursachen und dadurch die Zuverlässigkeit der Fremdstoffdetektion beeinträchtigt wird. Beleuchtungselemente mit einem breiteren Lichtspektrum wie Laser, Blitzlampen oder Glühlampen oder von einer LED-Multichipanordnung anstelle einer einzigen LED benötigen mehr Teile und erheblich größeren Bauraum. Bauraum steht jedoch bei derartigen Sensoren, wie zum Beispiel Garnsensoren, nur in sehr begrenztem Umfang zur Verfügung. Der Energieverbrauch einer beispielsweise alternativ zu einer einzigen LED eingesetzten Glühlampe ist deutlich höher.

Die DE 37 06 056 A1 offenbart zur Erzeugung und Erkennung von Farben an bewegten und ruhenden Medien eine Strahlungseinrichtung mit mindestens zwei, vorzugsweise jedoch drei Halbleiterstrahlern, wie LED oder Laser, die das Medium mit einer Strahlung beaufschlagt. Dadurch wird eine Mischstrahlung von Farben erzeugt, mit der die Detektierung unterschiedlicher Farben möglich ist. Dabei kann mindestens einer der Strahlungssender eine Mehrfarb-LED sein, bei der unterschiedliche Wellenlängen des Lichts auswählbar sind. Es ist alternativ nur jeweils eine einzige Wellenlänge einstellbar. Die Steuerung erfolgt über eine relativ aufwendige elektrische Steuereinrichtung. Die Schaltung zur Steuerung umfaßt mindestens einen Impulsgenerator, einen Impulsdauereinsteller und einen Impulsverstärker. Laser oder mehrere LED erfordern, wie bereits oben angeführt, mehr Aufwand und nehmen insbesondere einen höheren Anteil des begrenzten Bauraums ein.

Die EP 0 399 945 A2 beschreibt ein Verfahren und eine Vorrichtung zum Erkennen von Fremdstoffen in textilem Fasermaterial mit mindestens zwei Lichtquellen, die das Fasermaterial abwechselnd mit unterschiedlicher Farbe beleuchten. Dabei ist ein Sensor eingesetzt, der im Multiplexbetrieb abwechselnd beide Farben des vom Fasermaterial reflektierten Lichtes empfängt. Zur Erzeugung unterschiedlicher Farben kann eine Zweifarben-LED eingesetzt werden. Auch hier werden entweder mindestens zwei LED mit dem vorgenannten nachteiligen Raumbedarf oder bei einer Zweifarben-LED eine aufwendige Steuerung benötigt.

Die WO 98/33061 A1 beschreibt den Einsatz von unterschiedlichen Farben oder Wellenlängen. Unterschiedliche Farben oder Wellenlängen sollen verhindern, daß kontaminierende Fremdkörper im Prüfgut nur schwer oder gar nicht detektiert werden können. Auch der WO 98/33061 A1 ist zu entnehmen, daß es vorkommen kann, daß die Lichtstärke einer einzelnen LED nicht zufriedenstellend ist und zur Verstärkung der emittierten Lichtmenge und damit zur Verstärkung des elektro-optischen Signals anstelle einer einzigen LED eine Mehrzahl von LED eingesetzt werden sollte. Der Einsatz einer Vielzahl von Beleuchtungselementen oder der Einsatz von Beleuchtungselementen, die Licht mit hoher Intensität emittieren wie zum Beispiel Lichtbogenlampen, erfordert, wie bereits oben ausgeführt, zusätzliche Bauteile und und einen erheblichen Teil des bei einem Garnsensor nur sehr begrenzt zur Verfügung stehenden Bauraumes. Die in der WO 98/33061 A1 beschriebene Vorrichtung arbeitet mit einer Messung der Gesamthelligkeit des reflektierten Lichtes.

Es ist Aufgabe der Erfindung, eine Vorrichtung zur Erkennung von Fremdstoffen in strangförmigem textilen Material durch Einsatz geeigneter Mittel zu verbessern.

Diese Aufgabe wird erfindungsgemäß durch eine Vorrichtung mit den Merkmalen des Anspruches 1 gelöst.

Vorteilhafte Ausgestaltungen der Erfindung sind Gegenstand der Unteransprüche.

Eine farbiges monochromes Licht erzeugende Leuchtdiode mit Frequenztransformator, die Weißlicht emittiert, wird als weißleuchtende Ein-Chip-LED bezeichnet und nachfolgend Weißlicht-LED genannt. Die Weißlicht-LED emittiert Licht mit einer wesentlich höheren Intensität als bisher bekannte LED. Gleichzeitig besitzt dieses Licht ein breites Emissionsspektrum. Auf zusätzliche Leuchtdioden zur Verstärkung des Lichtes kann verzichtet werden. Auch die Verwendung von Anordnungen zusätzlicher Beleuchtungselemente, die Licht anderer Farbe emittieren, erübrigt sich. Gegenüber herkömmlichen Beleuchtungselementen mit einem breiten Wellenlängenspektrum, wie zum Beispiel Glühlampen, beträgt die Lebensdauer einer Weißlicht-LED bis zum 20fachen der Lebensdauer einer Glühlampe. Die Weißlicht-LED benötigt wesentlich weniger Bauraum als Laser, Blitzlampen oder Glühlampen. Meßfehler, wie sie beim Einsatz von mehr als einer LED durch unterschiedlich schnell verlaufendes Nachlassen der Intensität, sogenannter Alterung, des jeweils von einer LED emittierten Lichtes entstehen, werden beim Einsatz einer einzigen Weißlicht-LED vermieden. Die Alterung einer einzigen eingesetzten Weißlicht-LED kann auf relativ einfache Weise durch eine Kompensationseinrichtung ausgeglichen werden, die eine Regelung des zugeführten Stromes vornimmt und die Lichtintensität konstant hält. Dazu wird die Intensität des von der Weißlicht-LED emittierten Lichtes durch einen Sensor überwacht.

Auch fabrikneue LED emittieren jeweils Licht unterschiedlicher Intensität. Um fehlerhaften Messungen vorzubeugen, werden LED nach Lichtintensität in Klassen selektiert und nur LED aus klassengleicher Intensität zusammen eingesetzt. Eine derartige aufwendige Selektion kann beim Einsatz einer Weißlicht-LED entfallen.

Die auftretenden Unterschiede im Licht beim Vergleich mehrerer Weißlicht-LED sind wesentlich geringer als bei herkömmlichen LED. Damit ist die Reproduzierbarkeit der Messungen beim Austausch einer LED verbessert. Bei einer Weißlicht-LED, deren lichterzeugende Leuchtdiode als farbiges monochromes Licht Blaulicht liefert, nähert sich das Wellenlängenspektrum des emittierten Lichtes der Zusammensetzung von Sonnenlicht, zum Beispiel hat es wie das Sonnenlicht einen ausgeprägten Anteil an Blaulicht, und ist daher für die Beurteilung von textilem Material besonders geeignet. Weißlicht-LED emittieren ein sehr homogenes Licht, das gut zu Meßzwecken einsetzbar ist.

Eine Weißlicht-LED, bei der die Leuchtdiode, der Frequenztransformator und eine Linse in einem gemeinsamen Gehäuse angeordnet sind, benötigt außerordentlich wenig Bauraum, läßt sich leicht ein- und ausbauen und die einzelnen Bauelemente sind vor mechanischer Einwirkung und Verschmutzung besonders gut geschützt. Ein Frequenztransformator aus einem Epoxydharzformstoff mit darin verteilten Leuchtstoffpigmenten läßt sich einfach und vielseitig ausbilden und positionieren.

Während Naturfarben einen weitgestreuten Reflexionsbereich aufweisen, trifft man bei Pigmentfarbstoffen die Reflexion fast einwelliger Strahlung an. Eine sichere Farberkennung und eine zuverlässige Messung sind nur durch Beaufschlagen mit Licht jeweils ganz bestimmter Wellenlänge möglich. Das Licht einer LED bestimmter Farbe und gegebenenfalls bei einer sogenannten Zweifarben-LED das Licht einer zusätzlichen LED in einer anderen bestimmten Farbe decken das erforderliche Wellenlängenspektrum für die Erkennung von Pigmentfarben nur unzureichend ab. Die Weißlicht-LED verbessert die Anregung der Reflexionsstrahlung bei Pigmentfarbstoffen. Dadurch wird der Einsatzbereich erweitert und die Farberkennung beziehungsweise die Messung verbessert.

Eine Messung der Differenz in der Intensität der Spektren von Rotlicht und Grünlicht, von Blaulicht und Grünlicht sowie von Rotlicht und Blaulicht, wie sie mit drei Farbstützpunkten möglich ist, läßt auftretende Abweichungen von Farbwerten sowie ein Abdriften beziehungsweise Wandern der Farbwerte erkennen. Gleichzeitig kann ein Summenhelligkeitssignal gebildet werden, das ebenfalls ausgewertet wird, um Abweichungen festzustellen.

Die detektierten Meßwerte werden auf Merkmale untersucht, die auf das Auftreten von Fremdstoffen wie Fremdfasern oder Schalenfragmenten schließen lassen. Fremdfasern sind zum Beispiel andersfarbig beziehungsweise reagieren anders auf Färbemittel oder weisen ein anderes Reflexionsverhalten der Oberfläche auf. Eine Farberkennung über mehr als zwei, vorzugsweise drei Farbstützpunkte ermöglicht eine wesentlich genauere Messung und Auswertung und ergibt eine genaue Farbauflösung und eine hohe Zuverlässigkeit des Meßergebnisses. Damit sind auch Farbänderungen erkennbar, die bisher im Toleranzbereich der Schwankungen der Meßwerte lagen und daher nicht als Farbänderung erkannt wurden. Mit der erfindungsgemäßen Vorrichtung kann auf Schwankungen sensitiver reagiert werden.

Weitere Einzelheiten der Erfindung sind den Darstellungen der Figuren entnehmbar.

Es zeigt:
- Fig. 1:: eine schematische Darstellung einer Meßvorrichtung zur Fremdkörpererkennung mit einer Weißlicht-LED, . Photosensoren und einer Auswerteeinrichtung,
- Fig. 2:: die Prinzipdarstellung einer Schaltung zur Auswertung der Meßergebnisse.

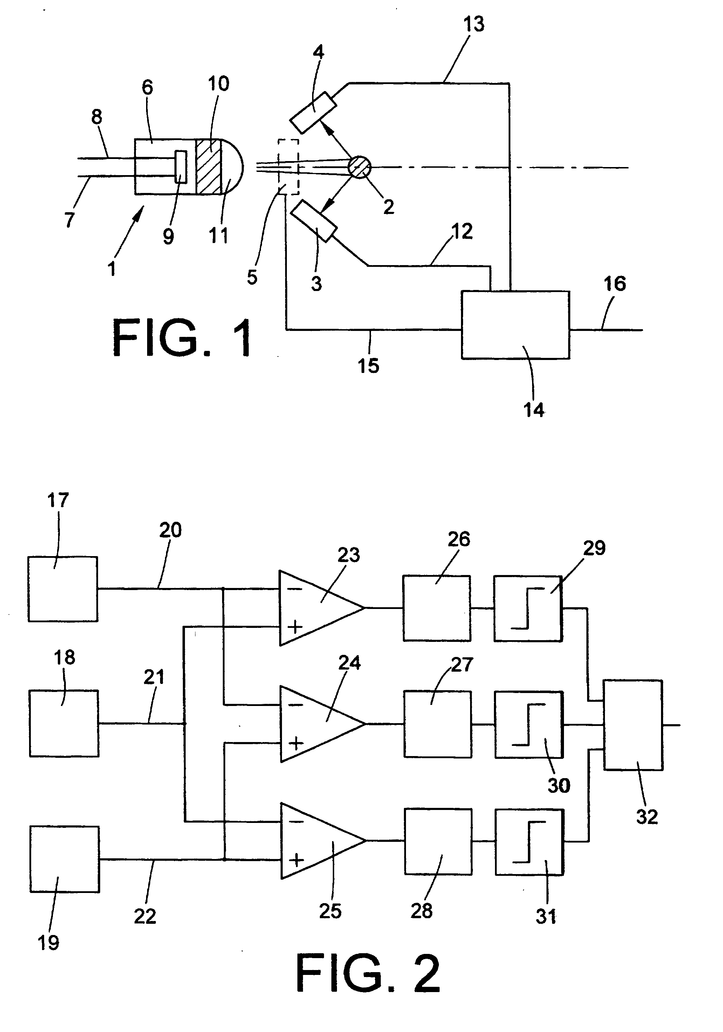

Die Meßeinrichtung der Fig. 1 weist eine Weißlicht-LED 1, einen Faden 2 und als Photosensor ausgebildete Sensoren 3,4,5 auf. Die Weißlicht-LED 1 besteht aus einer Baueinheit mit einem Gehäuse 6, einer über die Stromleiter 7,8 mit einer nicht dargestellten Spannungsquelle verbundenen, als LED-Chip ausgebildeten Leuchtdiode 9, einem Frequenztransformator 10 und einer Linse 11. Die Leuchtdiode 9 erzeugt einfarbiges Licht, im Ausführungsbeispiel blaues Licht, das vom Frequenztransformator 10 konvertiert und als Weißlicht mit einem breiten Wellenlängenspektrum, das aus einer Mischung von blauer Strahlung und gelber Konverterstrahlung besteht, durch die Linse 11 in Richtung des Fadens 2 emittiert wird. Der Frequenztransformator 10 besteht aus einem Epoxydharzformstoff mit darin verteilten Leuchtstoffpigmenten. Die Leuchtstoffpigmente sind in ihrer Zusammensetzung auf das von der Leuchtdiode 9 erzeugte Licht abgestimmt.

Das auf den Faden 2 auftreffende Licht wird von der Oberfläche des Fadens 2 reflektiert. Die Sensoren 3,4 erfassen das jeweils auf sie auftreffende Reflexlicht und wandeln es in ein der Reflexlichtstärke beziehungsweise der Reflexlichtintensität proportionales Signal um, das über die Leitungen 12,13 einer Signalverarbeitungsanlage 14 zugeführt wird. Die Signalverarbeitungsanlage 14 wertet die eingehenden Signale aus. Die Auswertung basiert im wesentlichen auf den gemessenen Intensitätswerten. Veränderungen der Intensität des Reflexlichtes beziehungsweise das Überschreiten mindestens eines Schwellwertes lassen darauf schließen, daß Fremdkörper im Faden 2 vorliegen.

Eine verbesserte Farberkennung wird durch den Einsatz eines dritten als Photosensor ausgebildeten Sensors 5 erzielt, der in der Fig. 1 gestrichelt dargestellt und über die Leitung 15 ebenfalls mit der Signalverarbeitungsanlage 14 verbunden ist. Der Sensor 5 ist etwas tiefer angeordnet als die Sensoren 3,4, damit er nicht den Strahlengang des Lichtes von der Weißlicht-LED 1 zum Faden 2 behindert. Die Leitung 16 dient zur Kommunikation der Signalverarbeitungsanlage 14 mit Datenverarbeitungs- oder anderen Signalverarbeitungsanlagen oder zur Steuerung eines nicht dargestellten Garnreinigers.

Mit der in Fig. 2 dargestellten Schaltung werden die Signale von drei Sensoren 17,18,19 ausgewertet, wobei die drei Sensoren 17,18,19 in unterschiedlichen Bereichen spektrale Empfindlichkeit aufweisen. Im Ausführungsbeispiel der Fig. 2 liegt die spektrale Empfindlichkeit des Sensors 17 im Bereich Rotlicht, des Sensors 18 im Bereich Grünlicht und des Sensors 19 im Bereich Blaulicht. Den Sensoren 17,18,19 folgt eine Subtraktionsstufe. Das von dem jeweiligen Sensor 17,18,19 emittierte Farbsignal wird über die zugeordnete Leitung 20,21,22 den Differenzsignalelementen 23,24,25 zugeführt. Dabei ist der Sensor 17 über die Leitung 20 mit den Differenzsignalelementen 23 und 24, der Sensor 18 über die Leitung 21 mit den Differenzsignalelementen 23 und 25 und der Sensor 19 über die Leitung 22 mit den Differenzsignalelementen 24 und 25 verbunden. Das Differenzsignalelement 23 empfängt ein Farbsignal vom Sensor 17, das proportional der Intensität des detektierten Rotlichtes ist sowie ein Farbsignal vom Sensor 18, das proportional der Intensität des detektierten Grünlichtes ist. Die Differenz in der Lichtintensität von Rotlicht und Grünlicht wird ermittelt und als Farbdifferenzsignal dem Hochpassfilter 26 zugeleitet. Der Hochpassfilter 26 läßt hochfrequente Farbdifferenzsignale durch und sperrt niederfrequente Farbdifferenzsignale und dient somit der Unterdrückung konstanter oder niederfrequenter Differenzen.

Nach Passieren des Hochpassfilters 26 wird das Farbdifferenzsignal in einem Komparator 29 daraufhin überprüft, ob ein vorgegebener, einstellbarer Schwellwert überschritten ist. Ist dies der Fall, wird ein Fehlersignal generiert und dem Schaltelement 32 zugeleitet. Die Messung der Differenz zwischen der Intensität der Farbsignale von Rotlicht und Blaulicht sowie von Blaulicht und Grünlicht erfolgt gleichzeitig und auf gleiche Weise wie die Messung der Differenz der Intensität der Farbsignale von Rotlicht und Grünlicht.

Das Schaltelement 32 enthält eine Oder-Schaltung und überprüft, ob vom Komparator 29 oder vom Komparator 30 oder vom Komparator 31 ein Fehlersignal vorliegt. Liegt das Fehlersignal von mindestens einem Komparator 29,30,31 vor, wird ein Fremdkörpersignal generiert. Die vom Schaltelement 32 ausgeführte Funktion wird auch als Oderierung der Fehlersignale zu einem Fremdstoffsignal bezeichnet. Bei Vorliegen eines Fremdstoffsignals kann, zum Beispiel durch die Signalverarbeitungsanlage 14 über die Leitung 10, die Schneidvorrichtung des Garnreinigers aktiviert und/oder andere geeignete Maßnahmen zur Aufrechterhaltung der gewünschten Garnqualität eingeleitet werden.

Weitere hier nicht erläuterte Einzelheiten von Aufbau und Wirkungsweise weißleuchtender Ein-Chip-LED können der Veröffentlichung "Langlebige Beleuchtung mit hohem Wirkungsgrad" aus Components 5/98 entnommen werden.

## Patentansprüche

1. Vorrichtung zur Erkennung von Fremdstoffen in strangförmigem textilen Material wie Faserbändern oder Garn, mit Mitteln zur Lichtgenerierung und zur Beaufschlagung des Materials mit Licht, zur Reflexlichtmessung sowie zur Auswertung,
**dadurch gekennzeichnet,**
**daß** das Mittel zur Lichtgenerierung und zur Beaufschlagung des Materials mit Licht eine einzige, monochromes blaues Licht erzeugende Leuchtdiode (9) und einen Frequenztransformator (10) aufweist, der so ausgebildet ist, daß mit ihm das blaue Licht in ein emittiertes Licht umwandelbar ist, das ein Weißlicht mit einem Wellenlängenspektrum ist, das dem Sonnenlicht gleichkommt.

2. Vorrichtung nach Anspruch 1, **dadurch gekennzeichnet, daß** die Leuchtdiode (1), der Frequenztransformator (10) und eine Linse (11) Teil einer Weißlicht-LED (1) und in einem gemeinsamen Gehäuse (6) angeordnet sind.

3. Vorrichtung nach Anspruch 1 oder 2, **dadurch gekennzeichnet, daß** der Frequenztransformator (10) ein Fluoreszenzmittel ist.

4. Vorrichtung nach Anspruch 1 oder 2, **dadurch gekennzeichnet, daß** der Frequenztransformator (10) aus einem Epoxydharzformstoff mit darin verteilten Leuchtstoffpigmenten besteht.

5. Vorrichtung nach einem der vorhergehenden Ansprüche, **dadurch gekennzeichnet, daß** die Mittel zur Reflexlichtmessung aus mehr als zwei Sensoren (3,4,5; 17,18,19) gebildet sind.

6. Vorrichtung nach einem der vorhergehenden Ansprüche, **dadurch gekennzeichnet, daß** die Mittel (3,4,5; 17,18,19) zur Reflexlichtmessung zur Farberkennung ausgebildet sind und die Farberkennung über mehr als zwei Farbstützpunkte erfolgt.

## Claims

1. An apparatus for detecting foreign substances in a strand-shaped textile material such as slivers or yarn, having means for light generation and for exposing the material to light, for the measurement of reflected light and also for evaluation,
**characterised in that** the means for the light generation and for exposing the material to light comprises a single light-emitting diode (9) which generates monochromatic blue light and a frequency transformer (10) which is constructed so that with it the blue light can be converted into an emitted light which is a white light having a wavelength spectrum equivalent to sunlight.

2. An apparatus according to Claim 1,
**characterised in that** the light-emitting diode (1), the frequency transformer (10) and a lens (11) are part of a white light LED (1) and are disposed in a common housing (6).

3. An apparatus according to Claim 1 or 2,
**characterised in that** the frequency transformer (10) is a fluorescent means.

4. An apparatus according to Claim 1 or 2,
**characterised in that** the frequency transformer (10) is made from an epoxy resin moulding material with luminescent pigments distributed therein.

5. An apparatus according to one of the preceding Claims,
**characterised in that** the means for the measurement of the reflected light are formed from more than two sensors (3, 4, 5; 17, 18, 19).

6. An apparatus according to one of the preceding Claims,
**characterised in that** the means (3, 4, 5; 17, 18, 19) for the measurement of the reflected light are constructed for colour recognition and colour recognition takes place via more than two colour support points.

## Revendications

1. Dispositif de détection de matières étrangères dans un matériau textile en écheveaux tel que rubans de fibres ou fil, avec des moyens de génération de lumière et d'exposition du matériau à la lumière, de mesure de la lumière réfléchie et d'évaluation,
**caractérisé par le fait**
**que** le moyen de génération de lumière et d'exposition du matériau à la lumière présente une diode électroluminescente (9) unique, produisant une lumière bleue monochromatique et un transformateur de fréquence (10) qui est conçu de manière à permettre la conversion de la lumière bleue en une lumière émise qui est une lumière blanche possédant un spectre de longueurs d'onde équivalent à celui de la lumière du soleil.

2. Dispositif selon la revendication 1, **caractérisé par le fait que** la diode électroluminescente (9), le transformateur de fréquence (10) et une lentille (11) font partie d'une DEL à lumière blanche (1) et sont disposés dans un boîtier commun.

3. Dispositif selon la revendication 1 ou 2, **caractérisé par le fait que** le transformateur de fréquence (10) est un produit fluorescent.

4. Dispositif selon la revendication 1 ou 2, **caractérisé par le fait que** le transformateur de fréquence (10) est constitué d'une matière à mouler en résine époxy dans laquelle sont répartis des pigments fluorescents.

5. Dispositif selon l'une des revendications précédentes, **caractérisé par le fait que** les moyens de mesure de la lumière réfléchie sont formés de plus de deux capteurs (3, 4, 5 ; 17, 18, 19).

6. Dispositif selon l'une des revendications précédentes, **caractérisé par le fait que** les moyens (3, 4, 5 ; 17, 18, 19) de mesure de la lumière réfléchie sont conçus pour la reconnaissance chromatique et que la reconnaissance chromatique est réalisée par plus de deux bases chromatiques.
